Europäisches Patentamt

European Patent Office

Office européen des brevets

(19)

(11) Veröffentlichungsnummer: **0 306 650 B1**

(12) **EUROPÄISCHE PATENTSCHRIFT**

(45) Veröffentlichungstag der Patentschrift: **27.11.91**

(51) Int. Cl.⁵: **C07H 15/04**

(21) Anmeldenummer: **88110838.5**

(22) Anmeldetag: **07.07.88**

(54) **Verfahren zur Herstellung von Alkyloligoglycosiden.**

(30) Priorität: **05.09.87 DE 3729842**

(43) Veröffentlichungstag der Anmeldung:
**15.03.89 Patentblatt 89/11**

(45) Bekanntmachung des Hinweises auf die
Patenterteilung:
**27.11.91 Patentblatt 91/48**

(84) Benannte Vertragsstaaten:
**BE DE ES FR GB IT NL**

(56) Entgegenhaltungen:
**EP-A- 0 165 721**
**FR-A- 1 549 814**
**US-A- 2 390 507**
**US-A- 3 219 656**

**THE MERCK INDEX, Ausgabe 10, 1983, Seiten
250-251, Ausgeber M. WINDHOLZ et al., Nr.
1792: "Carbon Amorphous", Merck & Co.,
Inc., Rahway, N.J., US**

**W. GARDNER: "Chemical Synonyms and Trade Names", Ausgabe 7, 1971, Seite 204, E.I.
COOKE, Technical Press, London, GB**

(73) Patentinhaber: **HÜLS AKTIENGESELLSCHAFT
- RSP Patente / PB 15 - Postfach 13 20
W-4370 Marl 1(DE)**

(72) Erfinder: **Lüders, Harald, Dr.
Langeoogstrasse 81
W-4350 Recklinghausen(DE)**

**Beschreibung**

Die Erfindung betrifft ein Verfahren, nach dem mit Hilfe von Adsorptionsmitteln hellfarbige Alkyloligoglycoside und Alkylglycoside, die Alkylgruppen mit 8 bis 24 C-Atomen aufweisen, hergestellt werden können.

Alkyloligoglycoside und Alkylglycoside mit $C_8$- bis $C_{24}$-Alkylresten können ganz oder teilweise aus nachwachsenden Rohstoffen hergestellt werden. Diese Alkyloligoglycoside und Alkylglycoside gewinnen wegen ihrer interessanten Tensideigenschaften bei gleichzeitig sehr guter biologischer Abbaubarkeit zunehmend an Bedeutung. Für Anwendungen im Haushalt und im Kosmetikbereich müssen diese Produkte hohen ästhetischen Ansprüchen genügen. Man ist daher an Verfahren interessiert, nach denen Alkyloligoglycoside und Alkylglycoside in transparenten, farblich schönen wäßrigen Lösungen hergestellt werden können.

Zur Herstellung von Alkyloligoglycosiden und Alkylglycosiden mit langkettigen Alkylgruppen stellt man im allgemeinen zunächst Alkyloligoglycoside und Alkylglycoside mit $C_1$- bis $C_6$-Alkylgruppen her. Diese Produkte werden dann mit langkettigen Alkoholen durch säurekatalysierte Umglycosidierung bei erhöhter Temperatur in die gewünschten Alkyloligoglycoside und Alkylglycoside übergeführt. Die so hergestellten Produkte sind jedoch dunkel gefärbt.

Nach EP 165 721 kann die Farbe derartiger Produkte durch mehrstufige Bleichung mit Wasserstoffperoxid verbessert und durch Zusatz von Schwefeldioxid freisetzenden Verbindungen stabilisiert werden.

Nach EP 77 167 können bei der Umsetzung von Alkoholen mit Aldosen oder Ketosen Reduktionsmittel wie hypophosphorige Säure oder schwefelige Säure zugesetzt werden. Dadurch wird die Farbe der Alkylglycoside verbessert.

Es sind auch vorbeugende Maßnahmen bekannt. So erhält man nach EP 102 558 farblich verbesserte $C_3$- bis $C_5$-Alkylglucoside, wenn man die Glucosidierung in Gegenwart eines Alkalisalzes einer Borsäure durchführt.

Bei der Herstellung von langkettigen Alkylsacchariden können nach US 4 465 828 auch die Hydroxypolycarbonsäuren Citronensäure, Weinsäure und Äpfelsäure zur Farbverbesserung beitragen.

Nach US 4 483 979 können Farbstoffe aus Alkylpolysacchariden extrahiert werden. Dieses aufwendige Verfahren erfordert wasserfreie, polare Lösemittel. Außerdem werden nach diesem Verfahren Teile der Alkylpolysaccharide mit extrahiert.

Die bekannten Verfahren zur Herstellung von farblich verbesserten Alkyloligoglycosiden sind umständlich, erfordern z. T. teure Chemikalien oder führen noch nicht zu den gewünschten Farbqualitäten.

Aufgabe der vorliegenden Erfindung ist es, ein vereinfachtes und in der Wirkung verbessertes Verfahren zur Herstellung von hellfarbigen Alkyloligoglycosiden und Alkylglycosiden mit $C_8$- bis $C_{24}$-Alkylgruppen bereitzustellen.

Die Aufgabe wird erfindungsgemäß dadurch gelöst, daß man bei der Herstellung der $C_8$- bis $C_{24}$-Alkyloligoglycoside und -Alkylglycoside

1. die Ausgangsverbindungen der Umglycosidierung in alkoholischen Lösungen oder die Produkte der Umglycosidierung in neutralen oder alkalischen alkoholischen Lösungen mit Aktivkohle behandelt,

2. das Lösemittel der Umglycosidierungsprodukte abdestilliert und

3. den Rückstand oder eine wäßrige Zubereitung davon mit Peroxidverbindungen bleicht.

Die Ausgangsverbindungen der Umglycosidierung sind $C_1$- bis $C_6$-Alkylglycoside und -Alkyloligoglycoside mit mittleren Oligomerisationsgraden von 1 bis etwa 4, die sich von Hexosen und Pentosen wie Glucose, Mannose, Galactose, Fructose, Xylose oder Arabinose herleiten lassen. Vorzugsweise sind es Butylglucoside und Butyloligoglucoside.

Die Umglycosidierung erfolgt im allgemeinen bei 80 bis 140 °C, vorzugsweise bei 90 bis 120 °C. Sie kann diskontinuierlich oder kontinuierlich durchgeführt werden. Sie wird nach einer (mittleren) Reaktionszeit von 0,5 bis 4 Stunden beendet.

Die Produkte der Umglycosidierung, die $C_8$- bis $C_{24}$-Alkyloligoglycoside und -Alkylglycoside, enthalten unverzweigte und verzweigte Alkylgruppen. Man kann sie durch Umglycosidierung mit linearen oder verzweigten primären Alkoholen und Alkoholgemischen mit 8 bis 24 C-Atomen herstellen. Vorzugsweise enthalten die Alkohole 8 bis 20 C-Atome.

Beispielsweise können native Tensidalkohole, wie sie unter anderem auch bei der Hydrierung von Fettsäuren oder Fettsäurederivaten entstehen, vollsynthetische Ziegleralkohole, Oxoalkohole oder Gemische davon eingesetzt werden.

Die Alkylglycoside und Alkyloligoglycoside weisen mittlere Oligomerisationsgrade von 1 bis etwa 10 auf und lassen sich von den gleichen Hexosen und Pentosen wie die Ausgangsverbindungen der Umglycosidierung herleiten. Vorzugsweise werden $C_8$- bis $C_{24}$-Alkylglucoside und -Alkyloligoglucoside hergestellt.

Entsprechend der vorliegenden Erfindung werden Produkte, deren 50 %ige wäßrige Lösungen eine Jodfarbzahl von unter 30 aufweisen, als hellfarbig bezeichnet.

Für die Ausgangsverbindungen der Umglycosidierung verwendet man als Lösemittel $C_1$- bis $C_6$-

Alkohole.

Lösemittel für die Produkte der Umglycosidierung sind unverzweigte oder verzweigté primäre Alkohole und Alkoholgemische mit 8 bis 24 C-Atomen.

Beispielsweise können native Tensidalkohole, vollsynthetische Ziegleralkohole und Oxoalkohole verwendet werden.

Für die Aktivkohlebehandlung können die bei der Glycosidierung anfallenden Lösungen von $C_1$- bis $C_6$-Alkyloligoglycosiden und -Alkylglycosiden in $C_1$- bis $C_6$-Alkoholen direkt eingesetzt werden.

Analog können auch die bei der Umglycosidierung anfallenden Lösungen von $C_8$- bis $C_{24}$-Alkyloligoglycosiden und -Alkylglycosiden in $C_8$- bis $C_{24}$-Alkoholen eingesetzt werden. In diesem Falle müssen die Lösungen jedoch vor der Aktivkohlebehandlung mit Alkali neutral oder alkalisch eingestellt werden.

Für die Aktivkohlebehandlung werden etwa 10 bis 90 %ige alkoholische Lösungen eingesetzt. Vorzugsweise verwendet man 15 bis 40 %ige Lösungen. Die Behandlung erfolgt bei 10 bis 140 °C, vorzugsweise bei 80 bis 130 °C. Dabei werden, bezogen auf die Lösung, 0,01 bis 10 % Aktivkohle mit der Lösung vermischt. Das Vermischen kann durch Rühren oder Schütteln erfolgen. Am Ende der Behandlung wird die Aktivkohle nach bekannten Methoden, wie beispielsweise Filtrieren oder Zentrifugieren, abgetrennt.

Bei kontinuierlicher Fahrweise kann die Lösung durch ein Aktivkohlebett, welches durch ein geeignetes Filter oder Sieb fixiert ist, gepumpt werden. Durch 1 kg Aktivkohle können bis zu etwa 10 000 kg Lösung verarbeitet werden.

Die Aktivkohle wird in handelsüblicher Qualität, beispielsweise in pulverisierter oder gekörnter Form, angewendet.

Um das Lösemittel abzutrennen, führt man vorzugsweise eine schonende Vakuumdestillation bei 100 bis 150 °C und einem Druck von 0,1 bis 10 mbar durch. Diese Destillation kann beispielsweise in einem Rotations- oder einem Dünnschichtverdampfer erfolgen.

Der Rückstand kann mit Wasser zu einer pumpbaren Lösung verarbeitet werden. Der Rückstand oder eine wäßrige Zubereitung wird mit Peroxidverbindungen wie Wasserstoffperoxid, Peressigsäure, Perbenzoesäure oder Peroxydischwefelsäure bei 50 bis 100 °C, vorzugsweise bei 70 bis 90 °C, gebleicht. Wasserstoffperoxid wird als Bleichmittel bevorzugt.

Durch das vorliegende Verfahren können hellfarbige $C_8$- bis $C_{24}$-Alkyloligoglycoside und -Alkylglycoside bei geringem apparativen Aufwand und geringem Aufwand an Chemikalien durch eine Adsorption und eine Bleichung hergestellt werden. Das Verfahren liefert Produkte mit hoher Reinheit und guter Farbqualität, die für eine breite Anwendungspalette eingesetzt werden können. Bei Verwendung von Aktivkohle und Wasserstoffperoxid werden Produkte erhalten, die durch keine zusätzlichen Chemikalien belastet sind, da man die unlösliche Aktivkohle abtrennt und Wasserstoffperoxid zu Wasser abreagiert.

Die folgenden Beispiele sollen die Erfindung verdeutlichen.

Beispiel 1

Herstellung einer Alkyloligoglucosidlösung:

In einem 500 ml-Rührkolben mit Rückflußkühler und Wasserabscheider werden 40 g Glucosesirup (70 %ig in Wasser), 50 g Butylglucosid, 82 g n-Butanol und 1,1 ml 2 N Schwefelsäure bei 700 bis 1 000 mbar auf 110 °C zum Sieden unter Rückfluß erhitzt. Dabei wird Wasser abgeschieden. Nach 1,5 Stunden ist die Fehling-Probe auf Glucose negativ.

Die nun vorliegende Butyloligoglucosidlösung in n-Butanol wird mit 250 g ALFOL[R] 1012 (Gemisch aus etwa 85 % n-Decanol, 8,5 % n-Dodecanol und 6,5 % n-Tetradecanol der Firma Condea, D-2212 Brunsbüttel) und 8,9 ml 2 N butanolischer Schwefelsäure vermischt und im Wasserstrahlvakuum auf 110 °C erhitzt. Dabei wird n-Butanol durch eine kurze Kolonne und einen aufgesetzten absteigenden Kühler abdestilliert. Nach 2,5 Stunden ist die Umglucosidierung beendet. Nun liegt eine $C_{10}$- bis $C_{14}$-Alkyloligoglucosidlösung vor.

Reinigung der Alkyloligoglucosidlösung:

Die vorliegende Lösung wird mit 2 N wäßriger Natronlauge bei 80 °C neutralisiert. Anschließend werden 2 g Aktivkohle (Art. Nr. 31 616 von Firma Riedel de Haen, D-3016 Seeze) zugegeben und 1 Stunde bei 100 °C verrührt und dann abfiltriert. Anschließend wird der Alkohol bei 150 °C und 1 bis 2 mbar abdestilliert. Aus dem Rückstand wird mit gleichen Teilen Wasser eine 50 %ige wäßrige Alkyloligoglucosidlösung hergestellt.

100 g Lösung werden mit 6,7 ml 30 %iger Wasserstoffperoxidlösung 1 Stunde bei 80 °C gebleicht. Die Lösung weist danach eine Jodfarbzahl von 15 auf.
Das Produkt erhält man nach Destillation des Wassers als hellfarbige Masse.

Vergleichsbeispiel A

Es wird wie in Beispiel 1 verfahren. Es wird jedoch keine Aktivkohlebehandlung durchgeführt. Die Lösung weist eine Jodfarbzahl von 80 auf.

Vergleichsbeispiel B

Es wird wie in Beispiel 1 verfahren. Man destilliert jedoch zuerst den Alkohol ab und führt erst danach an einer 50 %igen wäßrigen Alkyloligoglucosidlösung eine Aktivkohlebehandlung (2 g Aktivkohle/100 °C/ 1 Stunde) durch.
Nach dem Bleichen mit Wasserstoffperoxid weist die Lösung eine Jodfarbzahl von 40 auf.

Beispiel 2

Man verfährt wie in Beispiel 1. Man führt die Aktivkohlebehandlung jedoch bereits an der Butyloligoglucosidlösung in n-Butanol durch.
Die Lösung weist eine Jodfarbzahl von 20 auf.

Vergleichsbeispiel C

Es wird wie in Beispiel 1 verfahren. Man führt die Aktivkohlebehandlung jedoch an der Mischung aus Butyloligoglucosidlösung in Butanol, ALFOL$^R$ 1012 und butanolischer 2 N Schwefelsäure durch.
Die Lösung weist eine Jodfarbzahl von 60 auf.

**Patentansprüche**

1. Verfahren zur Herstellung von $C_8$- bis $C_{24}$-Alkyloligoglycosiden und -Alkylglycosiden, deren 50%ige wäßrige Lösungen eine Jodfarbzahl von unter 30 aufweisen, durch Glycosidierung und Umglycosidierung bei erhöhten Temperaturen mit Hilfe von Adsorptionsmitteln, dadurch gekennzeichnet, daß man
   1. die Ausgangsverbindungen der Umglycosidierung in alkoholischen Lösungen oder die Produkte der Umglycosidierung in neutralen oder alkalischen alkoholischen Lösungen mit Aktivkohle behandelt,
   2. das Lösemittel der Umglycosidierungsprodukte destilliert und
   3. den Destillationsrückstand oder eine wäßrige Zubereitung desselben mit Peroxidverbindungen bleicht.

2. Verfahren nach Anspruch 1, dadurch gekennzeichnet, daß man als Ausgangsverbindungen $C_1$- bis $C_6$-Alkyloligoglycoside und -Alkylglycoside, gelöst in $C_1$- bis $C_6$-Alkoholen, mit Aktivkohle behandelt.

3. Verfahren nach Anspruch 2, dadurch gekennzeichnet, daß man Butyloligoglucoside und Butylglucoside, gelöst in n-Butanol, mit Aktivkohle behandelt.

4. Verfahren nach Anspruch 1, dadurch gekennzeichnet, daß man als Produkte $C_8$- bis $C_{24}$-Alkyloligoglycoside und -Alkylglycoside, gelöst in neutral oder alkalisch eingestellten $C_8$- bis $C_{24}$-Alkoholen, mit Aktivkohle behandelt.

5. Verfahren nach Anspruch 4, dadurch gekennzeichnet, daß man $C_8$- bis $C_{24}$-Alkyloligoglucoside und -Alkylglucoside mit Aktivkohle behandelt.

6. Verfahren nach Anspruch 1, dadurch gekennzeichnet, daß man 10 bis 90 %ige alkoholische Lösungen mit 0,01 bis 10 % Aktivkohle, bezogen auf die Lösung, behandelt.

7. Verfahren nach Anspruch 6, dadurch gekennzeichnet, daß man 15 bis 40 %ige alkoholische Lösungen behandelt.

8. Verfahren nach Anspruch 1, dadurch gekennzeichnet, daß man die Aktivkohlebehandlung bei 10 bis 140 °C durchführt.

9. Verfahren nach Anspruch 8, dadurch gekennzeichnet, daß die Temperatur 80 bis 130 °C beträgt.

10. Verfahren nach Anspruch 1, dadurch gekennzeichnet, daß man mit Wasserstoffperoxid bei 50 bis 100 °C bleicht.

11. Verfahren nach Anspruch 1, dadurch gekennzeichnet, daß die Umglycosidierung bei 80 bis 140 °C in 0,5 bis 4 Stunden durchgeführt wird.

**Claims**

1. A process for the preparation of $C_8$- to $C_{24}$-alkyloligoglycosides and -alkylglycosides whose 50% strength aqueous solutions have an iodine colour number of less than 30 by glycosation and transglycosation at elevated temperatures by means of adsorbents, characterised in that
   1. the starting compounds of the transglycosation are treated with activated carbon in alcoholic solutions or the products of transglycosation are treated with activated carbon in neutral or alkaline alcoholic solutions,
   2. the solvent of the transglycosation products is distilled and

3. the distillation residue or an aqueous preparation thereof is bleached using peroxide compounds.

2. A process according to claim 1, characterised in that $C_1$- to $C_6$-alkyloligoglycosides and -alkylglycosides dissolved in $C_1$- to $C_6$-alcohols are treated with activated carbon as starting compounds.

3. A process according to claim 2, characterised in that butyloligoglucosides and butylglucosides dissolved in n-butanol are treated with activated carbon.

4. A process according to claim 1, characterised in that $C_8$- to $C_{24}$-alkyloligoglycosides and -alkylglycosides dissolved in $C_8$- to $C_{24}$-alcohols made neutral or alkaline are treated with activated carbon as products.

5. A process according to claim 4, characterised in that $C_8$- to $C_{24}$-alkyloligoglucosides and -alkylglucosides are treated with activated carbon.

6. A process according to claim 1, characterised in that 10 to 90% strength alcoholic solutions are treated with 0.01 to 10% of activated carbon, relative to the solution.

7. A process according to claim 6, characterised in that 15 to 40% strength alcoholic solutions are treated.

8. A process according to claim 1, characterised in that the activated carbon treatment is carried out at 10 to 140° C.

9. A process according to claim 8, characterised in that the temperature is 80 to 130° C.

10. A process according to claim 1, characterised in that the bleaching is carried out with hydrogen peroxide at 50 to 100° C.

11. A process according to claim 1, characterised in that the transglycosation is carried out at 80 to 140° C for 0.5 to 4 hours.

**Revendications**

1. Procédé de préparation d'alkyl-oligo-glycosides et d'alkyl-glycosides comportant de 8 à 24 atomes de carbone et dont les solutions aqueuses à 50 % présentent un indice d'iode inférieur à 30, par glycosidylation et transglycosidylation à des températures élevées, à l'aide d'agents d'adsorption, caractérisé par le fait :

1. que l'on traite par du charbon actif les composés de départ de la trans-glycosidylation dans des solutions alcooliques ou que l'on traite par du charbon actif les produits de trans-glycosidylation dans des solutions alcooliques neutres ou alcalines.
2. que l'on distille le solvant des produits de transglycosidylation,

et

3. que l'on blanchit avec des peroxydes le résidu de distillation ou une préparation aqueuse de celui-ci.

2. Procédé selon la revendication 1, caractérisé par le fait que l'on traite par du charbon actif les composés de départ, à savoir des alkyl-oligo-glycosides et des alkyl-glycosides en $C_1$ à $C_6$, en solution dans des alcools comportant de 1 à 6 atomes de carbone.

3. Procédé selon la revendication 2, caractérisé par le fait que l'on traite par du charbon actif des butyl-oligo-glucosides et des butyl-glucosides en solution dans du butanol normal.

4. Procédé selon la revendication 1, caractérisé par le fait que l'on traite par du charbon actif les produits que sont des alkyl-oligo-glycosides et des alkyl-glycosides comportant de 8 à 24 atomes de carbone, ce en solution dans des alcools neutres ou alcalinisés comportant de 8 à 24 atomes de carbone.

5. Procédé selon la revendication 4, caractérisé par le fait que l'on traite par du charbon actif des alkyl-oligo-glucosides et des alkyl-glucosides en $C_8$ à $C_{24}$.

6. Procédé selon la revendication 1, caractérisé par le fait que l'on traite des solutions alcooliques d'une teneur de 10 à 90 %, par 0,01 à 10 % de charbon actif, relativement à la solution.

7. Procédé selon la revendication 6, caractérisé par le fait que l'on traite les solutions alcooliques à 15 - 40 %.

8. Procédé selon la revendication 1, caractérisé par le fait que l'on effectue le traitement par le charbon actif à une température de 10 à 140°C.

9. Procédé selon la revendication 8, caractérisé par le fait que la température est de 80 à 130°C.

10. Procédé selon la revendication 1, caractérisé par le fait que l'on effectue le blanchiment avec du peroxyde d'hydrogène à une température de 50 à 100°C.

11. Procédé selon la revendication 1, caractérisé par le fait que l'on effectue la transglycosidylation en une demi-heure à quatre heures à une température de 80 à 140°C.